# EUROPEAN PATENT APPLICATION

(11) **EP 3 771 467 A1**
(43) Date of publication of application: **03.02.2021**
(21) Application number: 19382654.2
(22) Date of filing: 30.07.2019
(51) Int. Cl.: A61K 38/07, A61P 9/14, C07K 5/11

(54) **SS-31 FOR THE PREVENTION AND/OR TREATMENT OF ANEURYSM**

(71) Applicant: Fundacio Institut de Recerca de l'Hospital de la Santa Creu i sant Pau, 08025 Barcelona (ES); Consejo Superior de Investigaciones Científicas (CSIC), 28006 Madrid (ES)
(72) Inventor: GALÁN ARROYO, María, 08025 Barcelona (ES); NAVAS MADROÑAL, Miquel, 08025 Barcelona (ES); RODRIGUEZ SINOVAS, M. Cristina, 08025 Barcelona (ES); MARTÍNEZ GONZÁLEZ, José, 08025 Barcelona (ES)
(74) Representative: Elzaburu S.L.P.

(57) **Abstract**

The present invention relates to SS-31 or composition comprising SS-31 for use in the treatment and/or prevention of aneurysm.

## Description

### FIELD OF INVENTION

The present invention belongs to the field of biomedicine and relates to the use of the Szeto-Schiller-31 (SS-31) peptide for the prevention and/or treatment of aneurysm.

### BACKGROUND OF THE INVENTION

The Szeto-Schiller (SS)-tetrapeptides belong to a family of aromatic cationic peptides that are selectively concentrated 1000-fold in the mitochondrial inner membrane. SS-31 and SS-20 are mitochondrial-targeted protective peptides (Szeto HH, Antioxid Redox Signal. 2008 Mar; 10(3):601-19). SS-31 (also known as elamipretide, MTP-131 and Bendavia, with PubChem CID 11764719 and with CAS 736992-21-5) is the tetrapeptide D-Arg-2',6'-dimethyltyrosine-Lys-Phe-NH2 which is thought to stabilize cardiolipin, improve electron transport and reduce the production of toxic reactive oxygen species. SS-31 has demonstrated effects in experimental models of ischemia-reperfusion damage, hypertension and atherosclerosis (Kloner et al, 2015, Ann Transl Med, 3(2): 20). The SS-20 peptide is the tetrapeptide Phe-D-Arg-Phe-Lys-NH2, (with PubChem CID 134687495 and CAS 2105938-11 -0, as acetate salt). SS-20 inhibits mitocondrial permeability transition in a similar way to SS-31, but only SS-31 was demonstrated to directly inhibit reactive oxygen species (ROS) production and lipid peroxidation (Zhao K et al. J Biol Chem. 2004; 279: 34682-34690).

WO2004070054 A2 discloses that SS-31 inhibits mitochondrial swelling and cytochrome c release and protects myocardial contractile force during ischemia-reperfusion, while SS-20 offered no protection when given after ischemia (Szeto HH, Antioxid Redox Signal. 2008 Mar; 10(3):601-19). WO2009108695 A2 discloses that SS-20 and SS-31 significantly improved renal mitochondrial respiration after ischemia. WO2011044044 A1 discloses that SS-31 ameliorates angiotensin II (Angll)-induced cardiac hypertrophy, fibrosis and diastolic dysfunction. WO2011019809 A1 discloses that SS-20 and SS-31 are useful in reducing body weight in diabetic rats and that both peptides have beneficial effects on lipid metabolism. Anderson EJ et al. and Carter et al. disclose that SS-31, but not SS-20, attenuates insulin resistance in humans and rodents maintained on a high fat diet (Anderson EJ et al. J Clin Invest. 2009 Mar;119(3):573-81; Carter EA et al. Int J Mol Med. 2011 Oct;28(4) :589-94).

An aneurysm is the enlargement of an artery caused by weakness of the arterial wall. Often there are no symptoms, but a ruptured aneurysm can lead to fatal complications. Most aneurysms do not show symptoms and are not dangerous. However, at their most severe stage, some can rupture, leading to life-threatening internal bleeding.

Aneurysms affect a variety of arteries. The most significant aneurysms affect the arteries supplying the brain and the heart. An aortic aneurysm affects the body's main artery. The aorta is the large artery that begins at the left ventricle of the heart and passes through the chest and abdominal cavities. The normal diameter of the aorta is between 2 and 3 centimeters (cm) but can bulge to beyond 5 cm with an aneurysm. The most common aneurysm of the aorta is an abdominal aortic aneurysm (AAA). This occurs in the part of the aorta that runs through the abdomen. Less commonly, a thoracic aortic aneurysm (TAA) can affect the part of the aorta running through the chest. TAA has a survival rate of 56 percent without treatment and 85 percent following surgery. It is a rare condition, as only 25 percent of aortic aneurysms occur in the chest. Further, often other aneurysms such as popliteal or femoral aneurysms and even TAA coexists with AAA.

AAA is a disease with a high rate of morbidity and mortality and a prevalence which, in men over 65 years old, can be as high as 8 percent. In this pathology, which is usually asymptomatic, there is an irreversible degeneration of the vascular wall that causes progressive dilation of the aorta and its eventual rupture (deadly in more than 80 % of cases). Among the most outstanding aspects of vascular remodeling in this pathology are inflammation, neovascularization, degradation of the components of the extracellular matrix by an increase in the activity of matrix metalloproteinases (MMPs) and of other proteases and death by apoptosis of vascular smooth muscle cells (CMLV).

There are currently no pharmacological strategies to limit the development of AAA. The only therapeutic measure available is surgical intervention (open surgery or endovascular) of those aneurysms with a high risk of rupture (aortic diameter > 5.5 cm). Although it has been suggested that statins, doxycycline, COX-2 inhibitors or the angiotensin converting enzyme, among others, could reduce the progression of AAA, none of them have conclusively demonstrated clinical benefit. Therefore, there is a need to develop new pharmacological tools for the treatment and prevention of this pathology.

### SUMMARY OF THE INVENTION

The present invention relates to the peptide D-Arg-2',6'-dimethyltyrosine-Lys-Phe-NH2 or to a composition comprising a therapeutically effective amount of said peptide for use in the treatment and/or prevention of aneurysm.

### DESCRIPTION OF THE INVENTION

The inventors have found that SS-31 but not SS-20 is useful in preventing the formation of aneurysms in a mouse model. SS-31, but not SS-20, was capable of inhibiting the development of aortic aneurysm and increasing the survival rate.

The present invention provides a new therapy for the treatment and prevention of aneurysm, providing a method for the treatment, prevention, regression or slowing down of the development of human aneurysmal disease that involves giving the individual a sufficient amount of SS-31 to reduce the vascular diameter or slow down its dilation and to decrease aortic degeneration.

As used herein, the term "effective amount" refers to a quantity of the SS-31 peptide sufficient to achieve a desired therapeutic and/or prophylactic effect, e.g.: an amount which results in the prevention of, or a decrease in, aneurysm or one or more symptoms associated with aneurysm or an amount necessary to achieve the desired therapeutic effect, which is an improvement in the phenotype of the aorta or any improvement, inhibition, mitigation or control of the presence, prevalence, severity, symptoms, etc. of the disease. In the context of therapeutic or prophylactic applications, the amount of a compound administered to the subject will depend on the type and severity of the disease and on the characteristics of the individual, such as general health, age, sex, body weight and tolerance to drugs. It will also depend on the degree, severity and type of disease. The dosing regimen should be adjusted to provide the optimal therapeutic response. The skilled artisan will be able to determine appropriate dosages depending on these and other factors. The therapeutically effective amount is also that in which any toxic or adverse effects are more than compensated for by the beneficial therapeutic effect. The compound can also be administered in combination with one or more additional therapeutic compounds. In the methods described herein, the peptide may be administered to a subject having one or more signs or symptoms of aneurysm, such as increased aortic vascular diameter, aortic vascular dilatation, aortic vascular remodeling or aortic degeneration. For example, a "therapeutically effective amount" of the peptide is meant as levels in which the physiological effects of an aneurysm are, at a minimum, ameliorated.

The peptide D-Arg-2',6'-dimethyltyrosine-Lys-Phe-NH2 is also referred to in this specification as SS-31.

As used herein, the term "prevention" or "preventing" refers to keeping a disease, disorder or condition from occurring in a subject. In some cases, the subject may be at risk for developing the disease but has not yet been diagnosed as having the disease. As used herein, the term "prevention" can be understood as stabilization of the pathology and improving the phenotype of the aorta. The expression "improving the phenotype of the aorta" refers to any phenotypic (anatomic) change in the aorta when there is an aneurysm, as a result of the use of the composition of the invention, in comparison with the phenotype (anatomy) of the aorta when the composition of the invention is not used. This improvement can be, for example, but without limitation, a reduction in the diameter of the aorta, or in the parameters of neovascularization, degradation of the components of the extracellular matrix or death of the CMLV or a reduction in the severity of the pathology, preferably according to the Manning scale.

As used herein, the term "treatment" or "treating" refers to inhibiting, preventing or arresting the development of a pathology (disease, disorder or condition) and/or causing the reduction, remission, or regression of a pathology. Those of skill in the art will understand that various methodologies and assays can be used to assess the development of a pathology, and similarly, various methodologies and assays may be used to assess the reduction, remission or regression of an aneurysm. In particular, the method can preferably be ultrasonography or ultrasound, as disclosed in Ann Intern Med. 2014;161(4): I-26. doi: 10.7326/P14-9028. In the present invention, the prevention of the development of aneurysm is related to the prevention of the increase of the aortic vascular diameter, the slow down of the aortic vascular dilatation or the limitation of aortic vascular remodeling and/or aortic degeneration.

As used herein, the term "aneurysm" refers to a condition where a vascular wall shows a region abnormally weak and forms an outward bulging, like a bubble or balloon. As an aneurysm increases in size, the risk of rupture increases, leading to uncontrolled bleeding. Although they may occur in any blood vessel, particularly lethal examples include aneurysms of the Circle of Willis in the brain, aortic aneurysms affecting the thoracic aorta, and abdominal aortic aneurysms.

The "aortic aneurysm" or "aneurysmal disease of the aorta" is the disease, usually asymptomatic, which leads to an abnormal widening of the aorta, with the aorta being the main artery that runs from the heart through the chest and abdomen. In this disease there is a degeneration of the arterial wall that causes progressive dilation of the aorta and its eventual rupture. Therefore, if an aneurysm grows, it can rupture and cause dangerous bleeding and even death. Preferably, aortic aneurysms are those expansions that result in an increase in external aortic diameter greater than or equal to 1.5 times the external diameter of the aorta of a healthy individual of the same species who does not have an aneurysm. The most prominent aspects of vascular remodeling that occurs in the presence of aneurysmal disease are, but are not limited to, degradation of extracellular matrix components by increased activity of MMPs, inflammation, neovascularization, and death by apoptosis of CMLVs. There are two types of aortic aneurysm, both included in the context of this invention: the thoracic aortic aneurysm, which occurs in the part of the aorta that passes through the chest, and the abdominal aortic aneurysm, which occurs in the part of the aorta that passes through the abdomen.

In a preferred embodiment of the present invention, the aneurysm is an aortic aneurysm. More preferably, the aneurysm is an abdominal aortic aneurysm.

In a preferred embodiment, the composition comprises at least one pharmaceutically acceptable excipient. In the composition of the invention, the peptide D-Arg-2',6'-dimethyltyrosine-Lys-Phe-NH2 may also be combined with an excipient, adjuvant and/or pharmaceutically acceptable carrier and formulated for proper administration. Appropriate formulations are, but are not limited to, solid forms (capsules, tablets, pills, tablets, etc.), semi-solid forms (powders, granulated forms, gels or hydrogels, creams, ointments, balsams, mousses, ointments, foams, lotions, etc.) or liquid forms (solutions, suspensions, emulsions, oils, liniments, syrups, serums, vaporizers, aerosols, etc.), or the peptide may be included in a sustained release system. In a preferred embodiment of the present invention, the composition further comprises at least one pharmaceutically acceptable carrier. The term "pharmaceutically acceptable" indicates that the substance or composition must be compatible chemically and/or toxicologically, with the other ingredients comprising a formulation, and/or the mammal being treated therewith.

The composition of the invention can be administered orally, subcutaneously, intramuscularly, intrathecally, intracranially, intraarterially, intravenously, intradermally, transdermally, topically, by inhalation, rectally, vaginally or by any other administration routes known by the skilled in the art. In a preferred embodiment, the composition of the invention is administered orally, intravenously, subcutaneously, intramuscularly or by inhalation. More preferably, it is administered orally or subcutaneously.

In another preferred embodiment, the composition of the invention is administered by means of an endovascular device.

The composition of the invention can comprise further active agents in addition to peptide D-Arg-2',6'-dimethyltyrosine-Lys-Phe-NH2. In a preferred embodiment, the composition further comprises at least one of the active agents selected from a hypolipemiant agent, an antihypertensive agent selected from at least one of a betablocker, an angiotensin-converting enzyme inhibitor, a calcium channel blocker, an angiotensin receptor blocker and a diuretic. In a preferred embodiment, the hypolipemiant agent is a statin, preferably selected from simvastatin, atorvastatin, rosuvastatin, lovastatin, pitavastatin and pravastatin; the betablockers is selected from propranolol, bisoprolol and ometoprolol; the angiotensin-converting enzyme inhibitor is selected from benazepril, zofenopril, perindopril, trandolapril, captopril, enalapril, lisinopril and ramipril; the calcium channel blocker is selected from amlodipine, aranidipine, azelnidipine, barnidipine, benidipine, cilnidipine, clevidipine, efonidipine, felodipine, isradipine, lacidipine, lercanidipine, manidipine, nicardipine, nifedipine, nilvadipine, nimodipine, nisoldipine, nitrendipine, pranidipine, fendiline, gallopamil, verapamil, diltiazem, mibefradil, bepridil, flunarizine and fluspirilene; the angiotensin receptor blocker is selected from losartan, candesartan, telmisartan, valsartan and fimasartan; and the diuretic is selected from furosemide, ethacrynic acid, torasemide, bendroflumethiazide, hydrochlorothiazide, acetazolamide and methazolamide.

Typical subjects that may be treated according to this aspect of the present invention include mammals such as human beings or domesticated animals. In a preferred embodiment of the present invention, the composition is for use in a human patient.

In a preferred embodiment of the present invention, the composition is for use in a patient subjected to an endovascular repair.

In a preferred embodiment of the present invention, the peptide D-Arg-2',6'-dimethyltyrosine-Lys-Phe-NH2 is in the form a pharmaceutically acceptable salt thereof, preferably in the form of the hydrochloride salt. The term "salts" includes derivatives of an active agent, where the active agent is modified by making acid addition salts or acid base. Preferably, salts are pharmaceutically acceptable salts. Such salts include, but are not limited to, addition salts of pharmaceutically acceptable acids, addition salts of pharmaceutically acceptable bases, addition salts of pharmaceutically acceptable metals, ammonium salts and alkylated ammonium salts. Acid addition salts include salts of inorganic acids and organic acids. Representative examples of suitable acids include hydrochloric, acetic, trichloroacetic, methanesulfonic, hydrobromic tosyl, hydroiodic, phosphoric, sulfuric, nitric and similar acids. The peptide D-Arg-2',6'-dimethyltyrosine-Lys-Phe-NH2 for use according to the present invention can be used as free base or as a pharmaceutically acceptable salt thereof, such as trifluoroacetate salt, acetate salt or hydrochloride salt. In a preferred embodiment of the present invention, the hydrochloride salt is used.

As used herein, the term "saline" refers to saline solution, i.e.: 0,9% NaCl solution commonly used in medicine.

### DESCRIPTION OF THE DRAWINGS

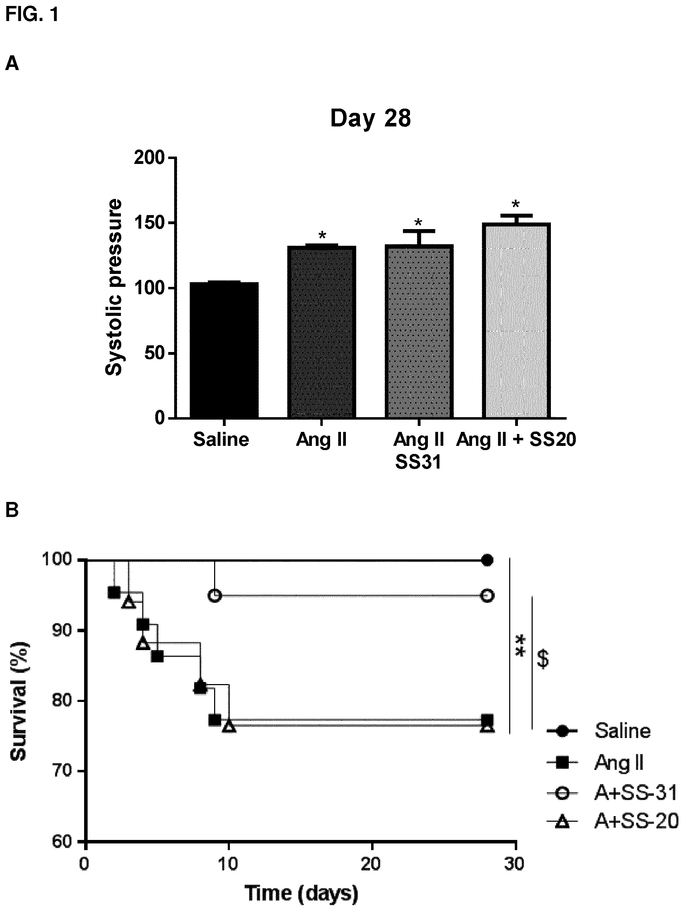

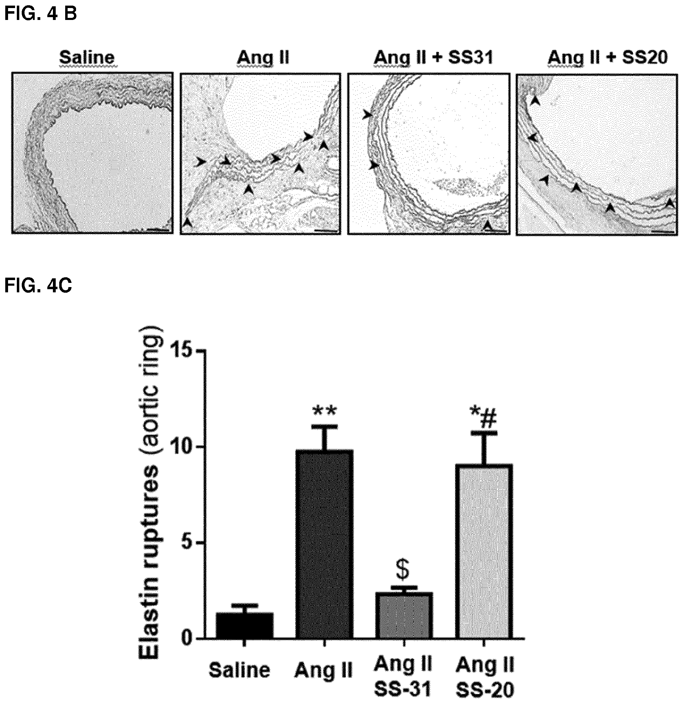

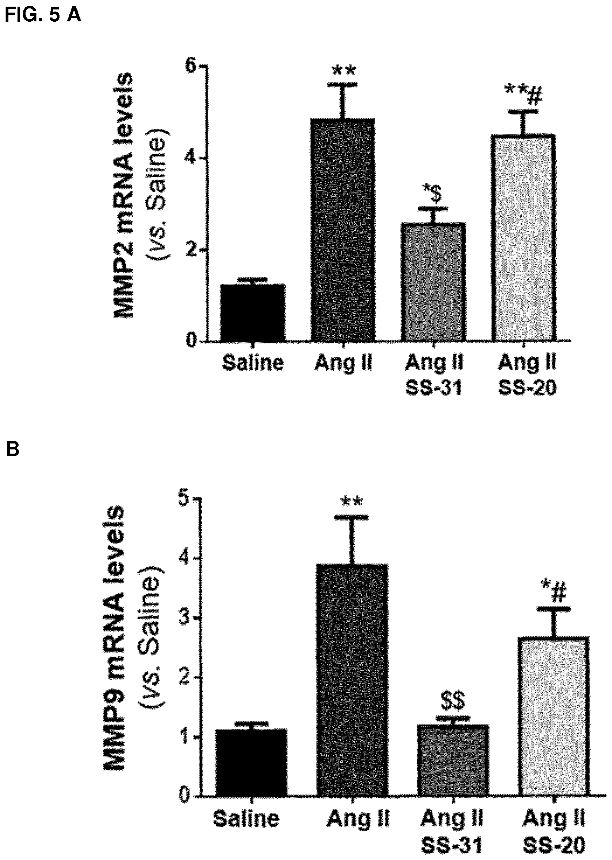

**Figure 1****.** Treatment with SS-20 or SS-31 does not affect systolic pressure and treatment with SS-31 improves survival in Apo E^{-/-} mice infused with saline or angiotensin II. A) Graph representing the systolic blood pressure of ApoE^{-/-} mice (males and females) infused with saline or angiotensin II (Ang II; 1000 ng/kg/min for 28 days) analyzed with the CODA® tail-cuff blood pressure system at the end of treatment. Animals infused with Ang II were treated or not with SS-31 or with SS-20 (3 mg/kg/day, administered together with Ang II through osmotic minipump). The values correspond to the mean ± SEM (n =7-14). *P < 0.05 vs. animals infused with saline. B) Percentage of animal survival in the four study groups described in (A). **P < 0.01 vs. saline at end time; $ P < 0.05 vs. animals infused with Ang II (not treated with SS-31) and treated or not with SS-20 at end time.

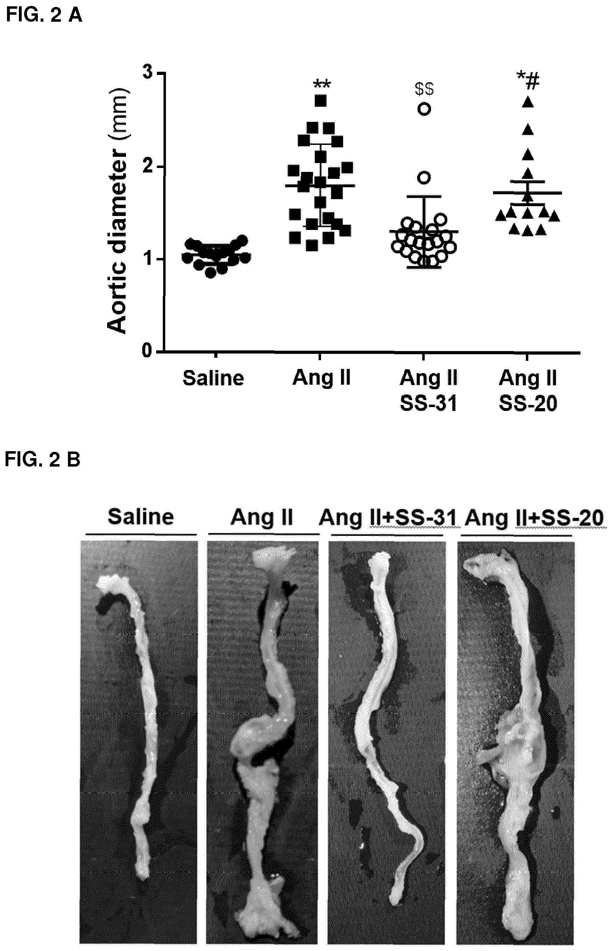

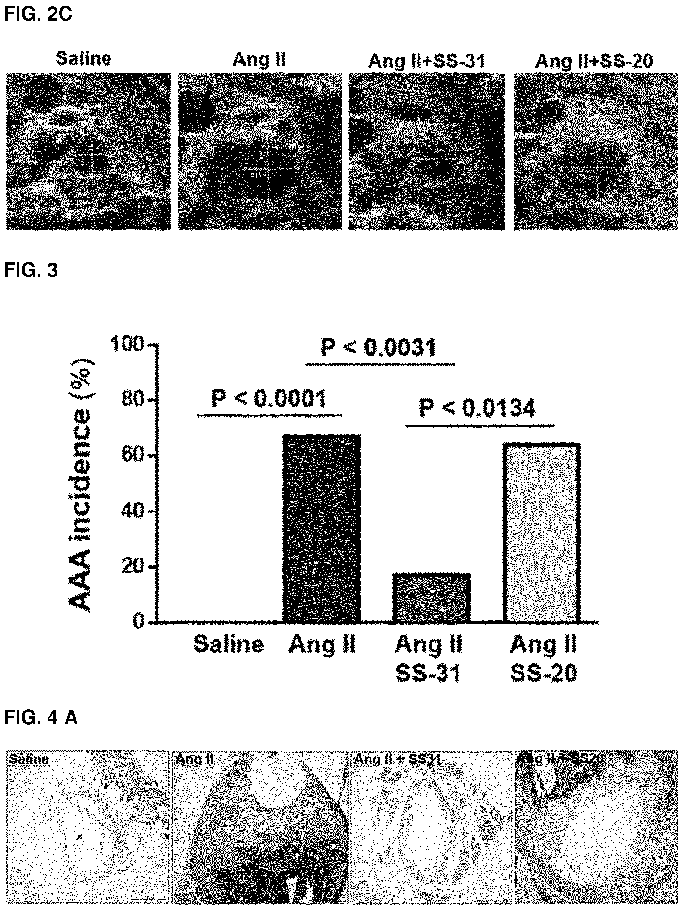

**Figure 2****.** Treatment with SS-31 inhibits the development of aortic aneurysm in angiotensin II-infused ApoE^{-/-} mice. A) Graphic representing the aortic diameter of ApoE^{-/-} mice infused with saline (Saline) or angiotensin II (Ang II; 1000 ng/kg/min for 28 days; males and females) analyzed by ultrasonography at the end of the study (28 days). Animals infused with Ang II were treated or not with SS-31 or with SS-20 (3 mg/kg/day, administered together with Ang II through osmotic minipumps). The values correspond to the mean ± SEM (n = 15-22). P < 0.01: ** vs. saline; $$ vs. animals infused with Ang II (not treated with SS-31 or SS-20); *P < 0.05 vs. saline; # P < 0.05 vs. animals infused with Ang II and treated with SS-31. B) Representative photographs of the aortas of the mice of the 4 study groups at the end of the study period (28 days). C) Representative images obtained by ultrasonography of the transversal vision of the aorta of each one of the experimental groups indicated in (A) at the end of the 4 weeks of treatment.
**Figure 3****.** Treatment with SS-31 decreases the incidence of AAA in ApoE^{-/-} mice infused with angiotensin II for 28 days. ApoE^{-/-} mice were infused with saline (Saline) or angiotensin II (Ang II; 1000 ng/kg/min for 28 days; males and females). Animals infused with Ang II were treated or not with SS-31 or with SS-20 (3 mg/kg/day, administered together with Ang II through osmotic minipumps). A representative graph of the incidence of aneurysm formation in the animals of the four study groups is shown.
**Figure 4****.** Treatment with SS-31 limits structural alterations in the abdominal aorta of ApoE^{-/-} mice infused with Ang II. ApoE^{-/-} mice were infused with saline (Saline) or Ang II (Ang II; 1000 ng/kg/min for 28 days; males and females). Animals infused with Ang II were treated or not with SS-31 or with SS-20 (3 mg/kg/day, administered together with Ang II through osmotic minipumps). (A and B) Representative images of histological analysis by Masson's trichrome (A) and orcein (B) staining in sections of the abdominal aorta of the indicated study groups. In (B) the elastic ruptures are indicated with arrows. Bar: 200 µm. (C) Histogram showing the quantification of the number of ruptures in the elastic fibers per aortic ring. The results are shown as mean ± SEM (n= 5; *P < 0.05, **P < 0.01 vs. Saline; $ P < 0.05 vs. animals infused with Ang II (not treated with SS-31 or SS-20); # P < 0.05 vs. animals infused with Ang II and treated with SS-31).
**Figure 5****.** Treatment with SS-31 reduces the expression of metalloproteinases MMP2 and MMP9 in the abdominal aorta of ApoE^{-/-} mice infused with angiotensin II. MMP2 and MMP9 expression levels analyzed by real-time PCR in the abdominal aorta of ApoE^{-/-} mice infused with saline (Saline) or angiotensin II (Ang II; 1000 ng/kg/min for 28 days; males and females) treated or not with SS-31 or with SS-20 (3 mg/kg/day, administered together with Ang II through osmotic minipumps). The results, normalized by the glyceraldehyde 3 phosphate dehydrogenase (GAPDH) mRNA level, are shown as mean ± SEM (n = 10-15; *P < 0.05, **P < 0.01,vs. Saline; $ P < 0.05, $$ P < 0.01 vs. Ang II (without additional treatments); # P < 0.01 vs. animals infused with Ang II and treated with SS-31).

### EXAMPLES

The invention is illustrated below by the examples showing the effectiveness of SS-31 treatment in reducing the incidence and severity of aortic aneurysms developed in response to Ang II infusion in Apo E^{-/-} mice (Daugherty A et al. J Clin Invest. 2000;105:1605-1612).

### Example 1: Analysis of the impact of SS-31 and SS-20 on the development of Ang II-induced aneurysms in ApoE^{-/-} mice.

The studies were developed in ApoE^{-/-} mice bred in the facility. The animals were infused with Ang II dissolved in saline (1000 ng/kg/min) by means of osmotic minipumps (model 1004, Alzet) implanted subcutaneously in the interscapular space of the mice previous anesthesia with isoflurane. The procedure lasted approximately 15 minutes per animal. Immediately after surgery, antibiotics (penicillin, 22,000 u/Kg, i.m.) and analgesics (buprenorphine 0.05 mg/Kg, i.m.) were administered to prevent infections and limit discomfort in the animals. Further, micewere kept on a heating pad until they woke up after surgery and were carefully monitored during the post-surgery period.

A group of mice infused with Ang II were treated with SS-31 in the form of chloride and dissolved in saline (3 mg/kg/day). Another group of animals infused with Ang II were treated with the SS-20 peptide also in the form of chloride, dissolved in saline and at the same dose. Both were administered together with Ang II by osmotic mini pump during the whole study (28 days). The control group consisted of ApoE^{-/-} mice infused with saline.

Blood pressure was evaluated at the end of treatment (28 days) using the tail plethysmography method (CODA®tail-cuff blood pressure system).

The diameter of the aorta was evaluated weekly by abdominal ecography using an ultrasound equipment (Vevo2100 Imaging systems; Visualsonics) with a 30 MHz transducer. The mice were anesthetized by inhalation of 1.5 % isofluorane and secured in supine position on a thermal platform. After shaving the precord region, the transducer was applied to the abdominal wall to measure the abdominal aorta. Those abdominal aortas with external diameters greater than or equal to 1.5 mm were considered aneurysmal. All measurements were made from the captured images using the analysis software provided by Visualsonics.

After 4 weeks, the animals were euthanized under intraperitoneal terminal anesthesia with a mixture of medetomidine (1 mg/kg) and ketamine (75 mg/kg) in saline (final volume of 200 µl), the aortas were collected immediately, examined to determine the presence of aneurysms and fixed in paraformaldehyde or frozen in liquid nitrogen for subsequent RNA extraction.

These analyses showed that infusion with Ang II increased blood pressure and that this parameter was not significantly altered as a consequence of treatment with SS-31 or SS-20 (Figure 1).

Infusion with Ang II caused the death of approximately 20 % of the animals, while treatment with SS-31 significantly improved the survival rate (Figure 1B). SS-20 did not modify the survival rate (Figure 1B).

Ultrasound studies showed that infusion with Ang II in ApoE^{-/-} mice significantly increased the diameter of the aorta compared to control animals infused with saline, and that this effect was attenuated by treatment with SS-31 (Figure 2A). The administration of SS-31 reduced the increase in aortic diameter significantly, as can also be determined through macroscopic visualization of the aorta (Figure 2B). However, SS-20 could not attenuate this increase in the aortic diameter (Figure 2B).

Accordingly, we observed that in animals infused with Ang II, treatment with SS-31 significantly decreased the incidence of AAA (Figure 3). On the contrary, the administration of SS-20 in animals infused with Ang II did not alter the aortic diameter nor the incidence of aneurysms, parameters that were similar to those of the group exclusively infused with Ang II (Figures 2 and 3).

Histological analyses using Masson's trichrome stain of sections of 5 µm of the fixed and paraffin-embedded aortic samples showed that in ApoE^{-/-} mice, infusion with Ang II produced an important vascular remodeling characterized by an increase in collagen deposition that was attenuated by treatment with SS-31 but not by treatment with SS-20 (Figure 4A). Likewise, orcein staining showed that SS-31 limited the disorganization and rupture of elastic fibers induced by Ang II infusion, an improvement in elastin structure that was not observed in animals treated with SS-20 (Figure 4B and 4C).

The expression levels (mRNA) of the metalloproteinases MMP2 and MMP9 in the abdominal aortas of the different study groups were also analyzed. For this purpose, the total RNA was extracted from the tissues with the RNeasy Micro kit (Qiagen) system following the manufacturer's instructions. The RNA (1 µg) was retrotranscribed to cDNA using the High Capacity cDNA Reverse Transcription Kit (Applied Biosystems) in the presence of random hexamers, and a real time PCR analysis was performed using specific Taqman® probes for MMP2 and MMP9. These analyses showed that Ang II-infusionin ApoE⁻¹⁻ mice significantly increased aortic expression of MMP2 and MMP9 with respect to control animals infused with saline solution. Treatment with SS-31 attenuated this effect, unlike SS-20, which did not modify the increase in the mRNA level of these MMPs induced by Ang II (Figure 5).

## Claims

1. The peptide D-Arg-2',6'-dimethyltyrosine-Lys-Phe-NH2 for use in the treatment and/or prevention of aneurysm.

2. The peptide according to the preceding claim, wherein the aneurysm is an aortic aneurysm.

3. The peptide according to any one of the preceding claims, wherein the aneurysm is an abdominal aortic aneurysm.

4. A composition comprising a therapeutically effective amount of peptide D-Arg-2',6'-dimethyltyrosine-Lys-Phe-NH2 for use in the treatment and/or prevention of aneurysm.

5. The composition according to the preceding claim, wherein the aneurysm is an aortic aneurysm.

6. The composition according to any one of the preceding claims, wherein the aneurysm is an abdominal aortic aneurysm.

7. The composition according to any one of the preceding claims, wherein said composition comprises at least one pharmaceutically acceptable excipient.

8. The composition according to any one of the preceding claims, wherein said composition is administered orally, intravenously, subcutaneously, intramuscularly or by inhalation.

9. The composition according to any one of the preceding claims, wherein said composition is administered by means of an endovascular device.

10. The composition according to any one of the preceding claims, further comprising at least one of the active agents selected from a hypolipemiant agent, an antihypertensive agent selected from at least one of a betablocker, an angiotensin-converting enzyme inhibitor, a calcium channel blocker, an angiotensin receptor blocker and a diuretic.

11. The composition according to the preceding claim, wherein the hypolipemiant agent is a statin, preferably selected from simvastatin, atorvastatin, rosuvastatin, lovastatin, pitavastatin and pravastatin; the betablockers is selected from propranolol, bisoprolol and ometoprolol; the angiotensin-converting enzyme inhibitor is selected from benazepril, zofenopril, perindopril, trandolapril, captopril, enalapril, lisinopril and ramipril; the calcium channel blocker is selected from amlodipine, aranidipine, azelnidipine, barnidipine, benidipine, cilnidipine, clevidipine, efonidipine, felodipine, isradipine, lacidipine, lercanidipine, manidipine, nicardipine, nifedipine, nilvadipine, nimodipine, nisoldipine, nitrendipine, pranidipine, fendiline, gallopamil, verapamil, diltiazem, mibefradil, bepridil, flunarizine and fluspirilene; the angiotensin receptor blocker is selected from losartan, candesartan, telmisartan, valsartan and fimasartan; and the diuretic is selected from furosemide, ethacrynic acid, torasemide, bendroflumethiazide, hydrochlorothiazide, acetazolamide and methazolamide.

12. The composition according to any one of the preceding claims, for use in a human patient.

13. The composition according to any one of the preceding claims, for use in a patient subjected to an endovascular repair.

14. The composition according to any one of the preceding claims, wherein the peptide is in the form a pharmaceutically acceptable salt thereof, preferably in the form of the hydrochloride salt.
